# EUROPEAN PATENT SPECIFICATION

(11) **EP 2 094 207 B1**
(45) Date of publication and mention of the grant of the patent: **09.09.2015**
(21) Application number: 07864354.1
(22) Date of filing: 13.11.2007
(51) Int. Cl.: A61B 18/04, A61F 7/12

(54) **HIGH PRESSURE AND HIGH TEMPERATURE VAPOR CATHETERS AND SYSTEMS**
KATHETER UND SYSTEME MIT HOCHDRUCK- UND HOCHTEMPERATURDAMPF
CATHÉTERS ET SYSTÈMES FONCTIONNANT À HAUTE PRESSION ET À VAPEUR HAUTE TEMPÉRATURE

(30) Priority: 13.11.2006 US 598362; 13.11.2006 US 598383
(43) Date of publication of application: 02.09.2009
(73) Proprietor: Uptake Medical Corp., Tustin, CA 92780 (US)
(72) Inventor: BARRY, Robert L., Kirkland, WA 98034 (US); CORCORAN, Dean, Bothell, WA 98012 (US); CRAN, Brian, Seattle, WA 98177 (US); HOEY, Michael, Shoreview, MN 55126 (US); LEE, Sheldon K., Seattle, WA 98101 (US); LYONS, Peter, Portland, OR 97215 (US)
(74) Representative: Price, Nigel John King
(86) International application number: PCT/US2007/084592
(87) International publication number: WO 2008/064026

(56) References cited:
- US-A- 5 562 608
- US-A1- 2005 222 485
- US-A1- 2006 135 955
- US-A1- 2006 161 233
- US-A1- 2006 224 154
- BECKER M D ET AL: "Lung volumes before and after lung volume reduction surgery", AMERICAN JOURNAL OF RESPIRATORY AND CRITICAL CARE MEDICINE, AMERICAN LUNG ASSOCIATION, NEW YORK, NY, US, vol. 157, 1 January 1998 (1998-01-01), pages 1593-1599, XP002967593, ISSN: 1073-449X

## Description

### FIELD OF THE INVENTION

This invention relates to medical systems, and in particular to intrabronchial systems for delivering a high pressure, high temperature vapor to one or more tissue targets in a patient's lungs.

### BACKGROUND OF THE INVENTION

Heating therapies are increasingly used in various medical disciplines including cardiology, dermatology, orthopedics, oncology as well as a number of other medical specialties. In general, the manifold clinical effects of superphysiological tissue temperatures results from underlying molecular and cellular responses, including expression of heat-shock proteins, cell death, protein denaturation, tissue coagulation and ablation. Associated with these heat-induced cellular alternations and responses are dramatic changes in tissue structure, function and properties that can be exploited for a desired therapeutic outcome such as tissue injury, shrinkage, modification, destruction and/or removal.

Heating techniques in the lung pose several technical challenges because lung tissue is more aerated than most tissues and also due to its vascularization. Accordingly, these new heating methods, devices and systems for rapid, controllable, effective and efficient heating of lung tissue are needed. The present invention is directed at meeting these as well as other needs.

US 2006/224154 A1 discloses an apparatus for the application of internal energies in an introduced media-tissue. The apparatus provides a fluid-carrying chamber in the interior of the device or working end. A source provides liquid media to the interior chamber wherein energy is applied to instantly vaporize the media. In the process of the liquid-to-vapor phase transition of a saline media in the interior of the working end, large amounts of energy are added to overcome the cohesive forces between molecules in the liquid, and an additional amount of energy is requires to expand the liquid 1000+ percent (PAD) into a resulting vapor phase. Conversely, in the vapor-to-liquid transition, such energy will be released at the phase transitions at the targeted tissue interface. That is, the heat of vaporization is released in tissue when the media transitioning from gaseous phase to liquid phase wherein the random, disordered motion of molecules in the vapor regain cohesion to convert to a liquid media. This release of energy (defined as the capacity for doing work) relating to intermolecular attractive forces is transformed into therapeutic heat for a thermotherapy within a targeted body structure.

US 2006/135955 A1 discloses a surgical system for applying energy to tissue. The system includes a probe having a handle end that extends to a working end; a source of a vaporized fluid media that communicates with at least one vapor outlet in the working end; and a negative pressure source that communicates with at least one aspiration port in the working end.

US 2006/161233 A1 discloses a device for delivering a thermal damaging agent to a targeted region of the patient's lung to raise the temperature of the lung tissue in the targeted region sufficiently high to render the targeted region essentially non-functional wherein neither blood flow or air flow occurs within the region. The device for delivering a thermal damaging agent includes an elongate shaft having a proximal portion, a distal portion, and a thermal damaging agent delivering lumen extending within at least a distal portion of the shaft. The device has at least one discharge port in the distal portion of the elongate shaft in fluid communication with the thermal damaging agent delivering inner lumen. A thermal damaging agent generator is in fluid communication with the thermal damaging agent delivery lumen in the elongate shaft and is configured for generating a thermal damaging agent at a temperature above 40°C to the tissue at the targeted region to render the region essentially non-functional. Preferably the device also includes an occluding member disposed on a distal portion of the shaft to occlude the airway passage proximal to the delivery location of the thermal damaging agent.

### SUMMARY OF THE INVENTION

According to the present invention there is provided the patient lung volume reduction system claimed in the accompanying claim 1. Additional aspects of the system are set out in the dependent claims.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** illustrates a human respiratory system;
**FIG. 2** illustrates the airway in the respiratory system;
**FIG. 3** illustrates one method of treating a volume of lung tissue in accordance with the present invention;
**FIG. 4** is a schematic illustrating one embodiment of a vapor generator in accordance with the present invention;
**FIG. 5** illustrates one embodiment of a generator display or user interface;
**FIG. 6** is a perspective view of one embodiment of an energy delivery catheter in accordance with present invention;
**FIG. 7** is a longitudinal cross-sectional view of yet another embodiment of a catheter in accordance with the present invention;
**FIG. 7A** transverse a cross-sectional view of the catheter of **FIG. 7** taken along lines **7A-7A;** and
**FIG. 7B** is a transverse cross-sectional view of catheter illustrated in **FIG. 7** taken along lines **7B-7B.**

### DETAILED DESCRIPTION OF THE INVENTION

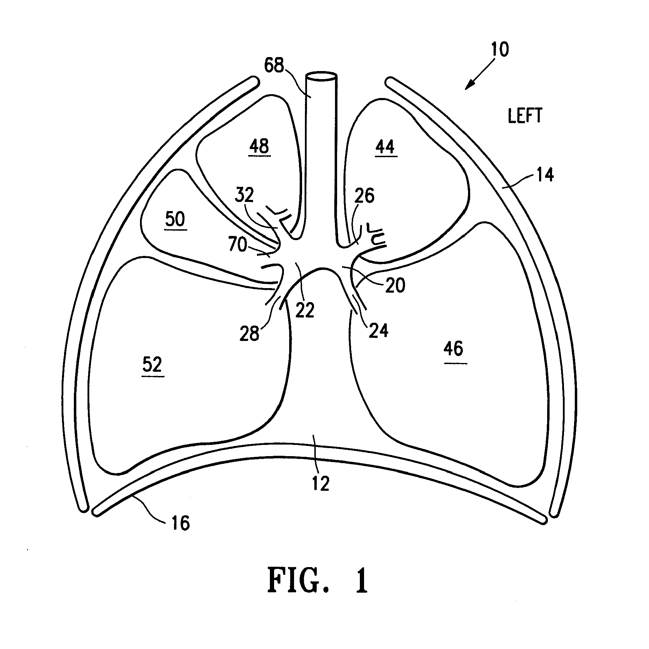

**FIG. 1** illustrates a human respiratory system **10.** The respiratory system **10** resides within the thorax **12** that occupies a space defined by the chest wall **14** and the diaphragm **16.** The human respiratory system **10** includes left lung lobes **44** and **46** and right lung lobes **48, 50,** and **52.**

The respiratory system **10** further includes trachea **18;** left and right main stem bronchus **20** and **22** (primary, or first generation) and lobar bronchial branches **24, 26, 28, 30,** and **32** (second generation). Segmental and sub-segmental branches further bifurcate off the lobar bronchial branches (third and fourth generation). Each bronchial branch and sub-branch communicates with a different portion of a lung lobe, either the entire lung lobe or a portion thereof. As used herein, the term "air passageway" or "airway" means a bronchial branch of any generation, including the bronchioles and terminal bronchioles.

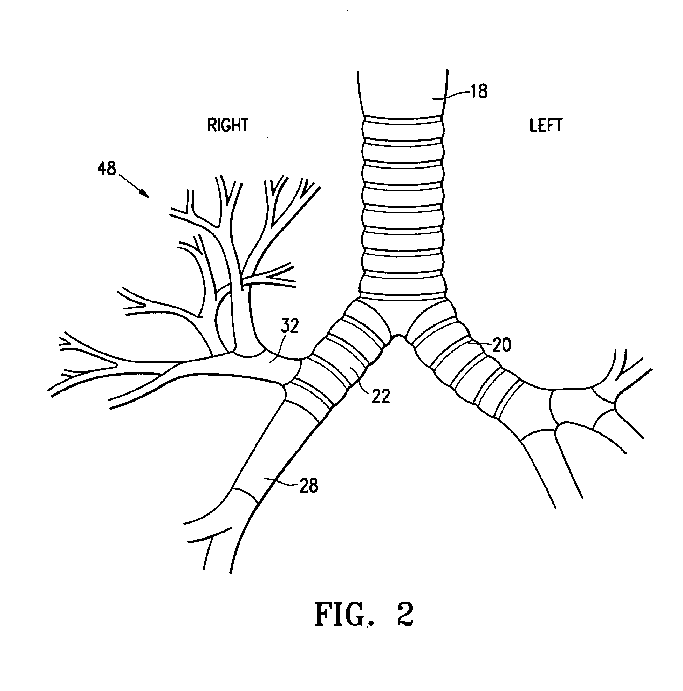

**FIG. 2** is a perspective view of the airway anatomy emphasizing the upper right lung lobe **48.** In addition to the bronchial branches illustrated in **FIG. 1****,** **FIG. 2** shows sub-segmental bronchial branches (fourth generation) that provide air circulation (i.e. ventilation) to superior right lung lobe **48.** The bronchial segments branch into six generations and the bronchioles branch into approximately another three to eight generations or orders. Each airway generation has a smaller diameter than its predecessor, with the inside diameter of a generation varying depending on the particular bronchial branch, and further varying between individuals. A typical lobar bronchus providing air circulation to the upper right upper lobe **48** has an internal diameter of approximately 1 cm. Typical segmental bronchi have internal diameter of approximately of about 4 to about 7 mm.

The airways of the lungs branch much like the roots of a tree and anatomically constitute an extensive network of air flow conduits that reach all lung areas and tissues. The airways have extensive branching that distally communicates with the parenchyma alveoli where gas exchange occurs. Because of these physiological characteristics of the airways, a medium, such as a vapor, delivered through an airway can be delivered focally or more regionally depending largely on the airway location at which the medium is delivered or dispersed.

While not illustrated, a clear, thin, shiny covering, known as the serous coat or pleura, covers the lungs. The inner, visceral layer of the pleura is attached to the lungs and the outer parietal layer is attached to the chest wall **14.** Both layers are held in place by a film of pleural fluid in a manner similar to two glass microscope slides that are wet and stuck together. Essentially, the pleural membrane around each lung forms a continuous sac that encloses the lung and also forms a lining for the thoracic cavity **12.** The space between the pleural membranes forming the lining of the thoracic cavity **12** and the pleural membranes enclosing the lungs is referred to as the pleural cavity. If the air tight seal around the lungs created by the pleural members are breached (via a puncture, tear, or is otherwise damaged) air can enter the sac and cause the lungs to collapse.

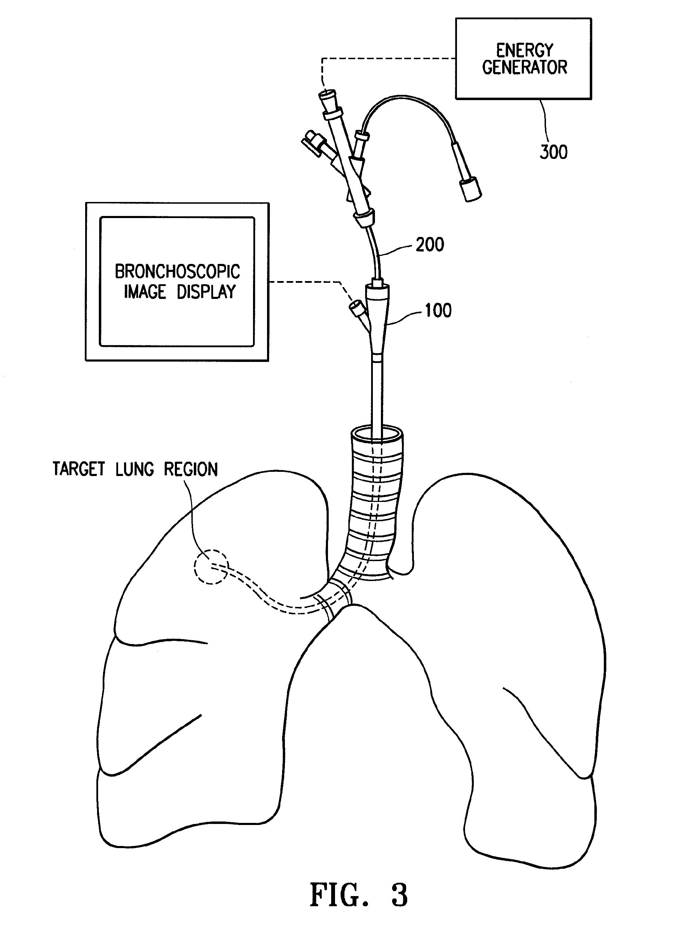

**FIG. 3** illustrates generally a procedure of relevance to the present invention. **FIG. 3** shows a bronchoscope **100** having a working channel into which an energy delivery catheter **200** is inserted. Bronchoscope **100** is inserted into a patient's lungs while the proximal portion of the energy delivery catheter **200** remaining outside of the patient. Energy delivery catheter **200** is adapted to operatively couple to an energy generator **300** as further discussed below.

Though not illustrated, patients can be intubated with a double-lumen endobronchial tube during the procedure, which allows for selective ventilation or deflation of the right and left lung. Depending on the location or locations of the target lung tissues to be treated, it may be preferable to stop ventilation of the target lung tissue. Also, while not illustrated, in an alternative embodiment, the procedure can be performed minimally invasively with energy catheter **200** introduced percutaneously through the chest wall and advanced to an appropriate location for with the aid of an introducer or guide sheath (with or without introduction into an airway).

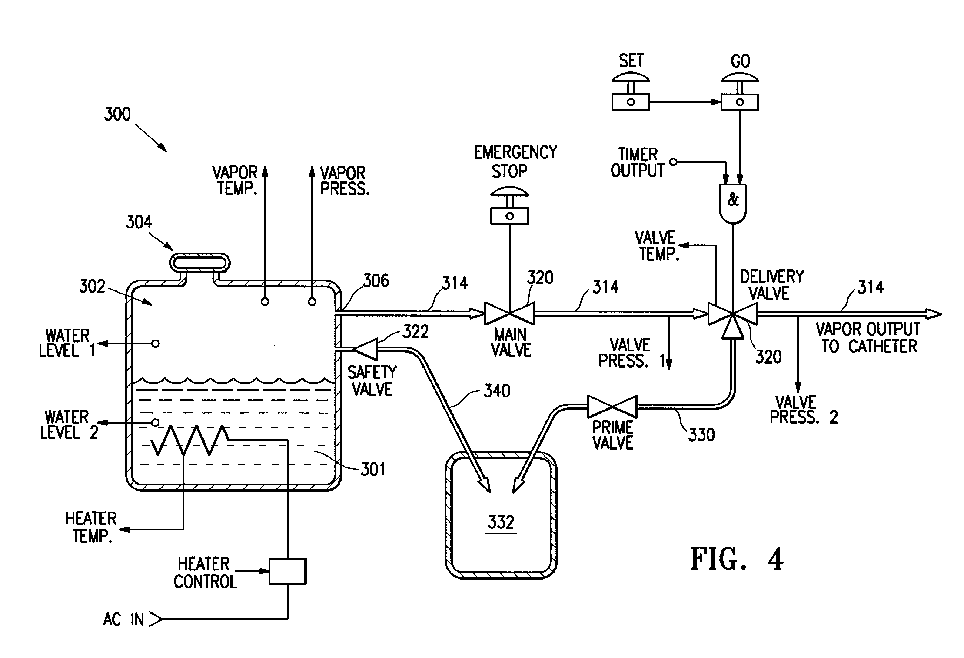

**FIG. 4** is a schematic diagram of one embodiment of the present invention wherein energy generator **300** is configured as a vapor generator. Preferably, vapor generator is configured to deliver a controlled dose of vapor to one or more target lung tissues. Generally, vapor generator **300** is adapted to convert a biocompatible liquid **301** (e.g. saline, sterile water or other biocompatible liquid), into a wet or dry vapor, which is then delivered to one or more target tissues. A wet vapor refers to a vapor that contains vaporous forms of the liquid as well as a non-negligible proportion of minute liquid droplets carried over with and held in suspension in the vapor. A dry vapor refers to a vapor contained little or no liquid droplets. In general, vapor generator **300** is configured to have a liquid capacity between about 1000 to 2500 cc and configured to generate a vapor having a pressure between about 5 - 50 psig (1.34 - 4.40 atm or 135799 - 446063 Pa) and temperatures between about 100 - 175° C.

Vapor generator **300** is preferably configured as a self-contained, medical-grade generator unit comprising at least a controller (not shown), a vaporizing unit **302,** a vapor inlet **304,** a vapor outlet **306** and a connective handle (not shown). The vaporizing unit **302** comprises a fluid chamber for containing a fluid **301,** preferably a biocompatible, sterile fluid, in a liquid state. Vapor outlet **304** is coupled to one or more pipes or tubes **314,** which in turn are in fluid communication with a vapor lumen of a hub assembly or other adapter, which in turn is adapted to operatively couple to the proximal end of energy delivery catheter **200,** several embodiments of which are further described below. Vapor flow from vapor generator **300** to a catheter (and specifically a vapor lumen of said catheter) is depicted as a vapor flow circuit **314** wherein flow of the vapor in circuit **314** is indicated by arrows **314** in **FIG. 4****.**

Vaporizer unit **302** is configured to heat and vaporize a liquid contained in a fluid chamber (not shown). Other components can be incorporated into the biocompatible liquid **301** or mixed into the vapor. For example, these components can be used in order to control perioperative and/or post procedural pain, enhance tissue fibrosis, and/or control infection. Other constituents, for the purpose of regulating vapor temperatures and thus control extent and speed of tissue heating, can be incorporated; for example, in one implementation, carbon dioxide, helium, other noble gases can be mixed with the vapor to decrease vapor temperatures.

Vaporizing unit **302** further comprises a fluid inlet **304** that is provided to allow liquid **301** to be added to the fluid chamber as needed. Fluid chamber can be configured to accommodate or vaporize sufficient liquid as need to apply vapor to one or more target tissues. Liquid in vaporizing unit **302** is heated and vaporized and the vapor flows into vapor outlet **306.** A number of hollow thermally conductive pipes **314** are adapted to fluidly connect vapor outlet **306** and handle, which in turn is adapted to operatively couple to a variety of energy delivery catheters, which are further described below. Preferably, there is little or no vapor-to-liquid transition during movement of the vapor through vapor flow circuit **314.** Vapor flow through vapor flow circuit **314** is unidirectional (in the direction of arrows **314**). Accordingly one or more isolation valves **320** are incorporated in vapor flow circuit **314.** Isolation valves **320,** which are normally open during use of generator **300,** minimize vapor flow in a direction opposite that of the vapor flow circuit **314.**

A priming line **330,** branching from main vapor flow circuit **314,** is provided to minimize or prevent undesirable liquid-state water formation during vapor flow through vapor flow circuit **314.** Pressure and temperature changes along vapor flow circuit **314** can affect whether the vapor is sustainable in a vapor state or condensed back into a liquid. Priming line **330** is provided to equalize temperatures and/or pressures along vapor flow circuit **314** in order to minimize or prevent undesirable liquid-state transition of the vapor during its progression through vapor flow circuit **314.** In one embodiment, an initial "purge" or "priming" procedure can be preformed prior to delivery of a therapeutic vapor dose in order to preheat flow, circuit **314** thus maintaining a constant temperature and pressure in the main vapor flow circuit **314** prior to delivery of a vapor to the target lung tissue.

As shown in **FIG. 4****,** priming line **330** terminates at evaporator **332,** which is adapted to either house undesirable liquid in a collection unit (not shown) located within generator **300.** In one embodiment, collection unit is adapted to house the liquid until a user or clinician is able to empty said collection unit. Alternatively, evaporator **332** is configured to evaporate and expel said undesirable liquid into the ambient air. Baffle plates (not shown) or other like means can be incorporated in evaporator **332** to facilitate maximal vapor-to-liquid transition. It should be understood that other suitable evaporator configurations could be included to facilitate vapor-to-liquid transition during a priming procedure of lines **314.**

A number of sensors, operatively connected to a controller, can be incorporated into vapor generator **300.** For example, in the liquid chamber, or along any point in vapor flow circuit **314,** a number of sensors can be provided. Water level sensors, adapted to monitor the water level in the liquid chamber, can be included. These water level sensors are configured as upper and lower security sensors to sense or indicate when a liquid level in the fluid chamber is below or above a set fluid level. In example, if a water level in the fluid chamber falls below the level of a lower water control sensor, the controller can be configured to interrupt the operation of the vapor generator **300.**

In yet another embodiment, pressure sensors, or manometers, can be included in vaporizing unit **302,** or at various points along the vapor flow circuit **314,** to measure the liquid or vapor pressures at various discrete locations and/or to measure vapor pressures within a defined segment along circuit **314.** One or more control valves **320** are installed at various points in the vapor flow circuit **314** to control vapor flow for instance to control or increase the vapor flow or vapor flow rates in vapor flow circuit **314.** In yet another embodiment, a safety valve **322** can be incorporated into the liquid chamber of vaporizing unit **302** and coupled to a vapor overflow line **340** if the need for removing or venting vaporizing unit **302** arises during generator **300** operation.

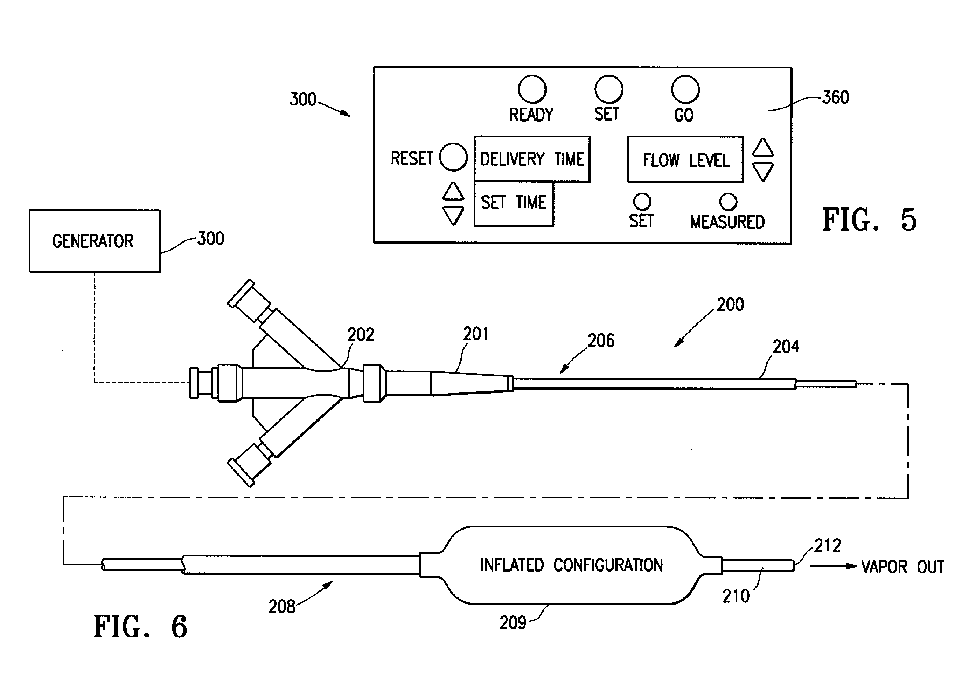

**FIG. 5** illustrates one embodiment of a user interface **360** of vapor generator **300.** As illustrated, the user interface **360** comprises various visual readouts intended to provide clinical users information about various treatment parameters of interest, such as pressure, temperature and/or duration of vapor delivery. Vapor generator **300** can also be adapted to incorporate one or more auditory alerts, in addition or in lieu of, visual indicators provided on user interface **360.** These one or more auditory alerts are designed to provide an alert to a clinical user, such as when vapor delivery is complete, when liquid chamber must be refilled or the like. As will be recognized by those in the art, other components, while not shown, can be incorporated including any of the following: a keyboard; a real-time imaging system display (such as a CT, fluoroscopy, ultrasound); memory system; and/or one or more recording systems.

**FIG. 6** illustrates yet another aspect of the invention, in particular a vapor catheter **200** embodying various features of the present invention. Generally, catheter **200** is adapted to operatively connect to a control handle of vapor generator **300** via hub assembly **202.** Catheter **200** includes elongate shaft **204** defined by proximal section **206** and distal section **208.** Elongated shaft **204** is formed with at least one lumen (such as a vapor, inflation, sensing, imaging, guide wire, vacuum lumen) extending from proximal section **206** to distal section **208** of shaft **204.** Starting at proximal section **206,** catheter **200** comprises strain relief member **201.**

Elongated shaft **204** further comprises at least one occlusive member **209** disposed at distal section **208** and distal tip **210** having at least one distal port **212.** In one embodiment, the at least one distal port **212** is configured as a vapor outlet port. In yet another embodiment, vapor outlet port may also be used as an aspiration port while catheter is coupled to a vacuum source (not shown) in order to aspirate mucus, fluids, and other debris from an airway through which catheter **200** is advanced prior to vapor delivery. Alternatively, catheter **200** can be configured to include a separate vacuum lumen and aspiration ports as needed. Distal tip **210** can be adapted into a variety of shapes depending on the specific clinical need and application. For example, distal tip **210** can be adapted to be atraumatic in order to minimize airway damage during delivery.

The dimensions of the catheter are determined largely by the size airway lumen through which the catheter must pass in order to deliver the catheter to an airway location appropriate for treatment of the one or more target tissues. An airway location appropriate for treatment of a target lung tissue depends on the volume of the target tissue and the proximity of catheter tip to the target tissue. Generally, catheter **200** is low profile to facilitate placement of catheter distal tip **210** as close as practicable to proximally and peripherally located target lung tissue, i.e. in order to facilitate the catheter's advancement into smaller and deeper airways. In addition, the low profile feature of catheter **200** also ensures that catheter can be delivered to the lungs and airways through a working channel of a bronchoscope, including for example, through the working channels of ultra-thin bronchoscopes. Preferably, catheter **200** is slidably advanced and retracted from a bronchoscope working channel. The overall length and diameter of catheter **200** can be varied and adapted according to: the specific clinical application; size of the airway to be navigated; and/or the location of the one or more target tissues.

Occlusive member or members **209** are similarly configured to provide the smallest possible size when deflated to facilitate ready retraction of catheter **200** back into the working channel of a bronchoscope following completion of a treatment procedure involving delivery of one or more vapor doses to one or more target tissues. The one or more occlusive members **209** are provided to obstruct of proximal vapor flow and/or seat catheter **200** in the patient's airway during vapor delivery without slipping.

Obstruction of an airway by occlusive member **209** prevents retrograde flow of vapor to tissues located outside of the desired target tissues. Because of the physiological characteristics of the airways, in particular the fact that the airways ventilate and communicate specific lung parenchyma or tissues, vapor delivered or dispersed at a particular airway location (e.g. at the bronchial, sub segmental, main bronchi) determines whether there is a focal or regional heating of tissue. In addition to location of the catheter distal tip, other considerations that impact whether there is focal or regional tissue heating patterns (i.e. volume of tissue heated or size of thermal lesion) created include: time or duration of vapor delivery; the vapor flow rate; and vapor content (dry vs. wet; vapor alone vs. vapor cocktail). Preferably, the one or more occlusive members **209** are compliant to ensure: adequate seating; airway obstruction; and/or complete collapse following deflation.

Catheter **200** can be fabricated from a variety of suitable materials and formed by any process such as extrusion, blow molding, or other methods well know in the art. In general, catheter **200** and its various components are fabricated from materials that are relatively flexible (for advancement into tortuous airways) yet having good pushability characteristics and durable enough to withstanding the high temperatures and pressures of the vapor delivered using catheter **200.**

Catheter **200** and elongated shaft **204** can be a tubular braided polyimide, silicone, or reinforced silicone. These materials are relatively flexible, yet have good pushability characteristics, while able to withstand the high temperature and pressures of vapor flow. Suitable materials should be adapted to withstand vapor pressures of up to 80 psig (6.444 atm), at temperatures up to 170°C. Specific suitable materials include, for example, various braided polyimide tubing available, for example, from IW High Performance Conductors, Inc. (See www.iwghpc.com/MedicalProducts/Tubing.html.) Similarly, the one or more occlusive members **209** are preferably fabricated from similar materials having pressure and temperature tolerant attributes as elongated shaft **204,** but preferably which is also compliant, such as silicone available from Dow Corning Q74720. As an added feature, catheter **200** and elongated shaft **204** can further be adapted to include varying flexibility and stiffness characteristics along the length of shaft **204** based on the clinical requirements and desired advantages. While not shown, various sensing members, including for example pressure, temperature and flow sensors known in the art can be incorporated into catheter **200.** For example, catheter **200** can be adapted to include a sensing lumen for advancement or connection with various sensory devices such as pressure, temperature and flow sensors.

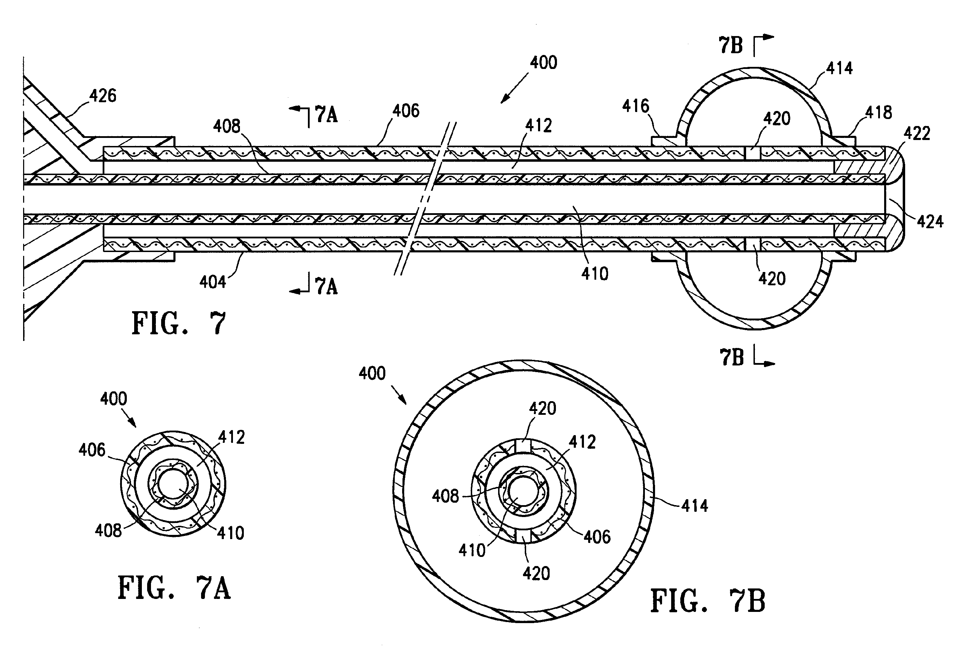

Turing now to **FIGS.7****,** illustrated is a preferred embodiment of a vapor catheter **400.** **FIG. 7** is a longitudinal cross sectional view of the elongate shaft **404** while **FIGS. 7A** and **7B** show transverse cross sectional views of the elongate shaft **404** taken along the lines **7A-7A** and lines **7B-7B** respectively.

In this preferred embodiment, catheter **400** comprises an elongated catheter shaft **404** having an outer tubular member **406** and an inner tubular member **408** disposed within outer tubular member **406.** Inner tubular member **408** defines a vapor lumen **410** adapted to receive a vapor and which is in fluid communication with a vapor flow circuit **314** of generator **300.** The coaxial relationship between outer tubular member **406** and inner tubular member **408** defines annular inflation lumen **412.** Vapor lumen **410** terminates at vapor port **424.**

Inflation balloon **414** is disposed on a distal section of elongated catheter shaft **404** and having proximal **416** and distal **418** balloon ends sealingly secured to outer tubular member **406.** One or more inflation ports **420** are disposed on outer tubular member **406** between the proximal **416** and distal **418** ends of inflation balloon **414** so that the interior of inflation balloon **414** is in fluid communication with inflation lumen **412.** (See **FIG. 7B****.**)

As shown in **FIG. 7****,** structural members **422** are disposed between inner tubular member **408** and outer tubular member **406** at distal vapor port **424** to seal inflation lumen **412** and provide structural integrity at the catheter tip. Structural members **422** are preferably made of stainless steel, nickel titanium alloys, gold, gold plated materials or other radiopaque materials, to provide catheter tip visibility under fluoroscopy and/or provide sufficient echogenicity so that the catheter tip is detectable using ultrasonography. Hub assembly **426** (or other adaptor) at the proximal end of catheter **400** is configured to direct an inflation fluid (such as a liquid or air) into inflation lumen **412** as well as provide access to vapor lumen **410.**

**FIG. 7B** illustrates inflation balloon **414** in an inflated or expanded configuration. Inflation balloon **414** inflates to a cylindrical cross section equal to that of a target airway in order to obstruct the airway and prevent proximal or retrograde vapor flow. This inflated configuration is achieved at an inflation pressure within the working pressure range of balloon **414.** Inflation balloon **414** has a working length, which is sufficiently long to provide adequate seating in a target airway without slippage during or prior to vapor delivery.

Suitable dimensions for the vapor catheter **400** in accordance with the present invention include an outer tubular member **406** which has an outer diameter of about 0.05 to about 0.16 inches (about 0.127 to about 0.4064 cm), usually about 0.065 inches (about 0.1651 cm) and an inner diameter of about 0.04 to about 0.15 inches (about 0.1016 to about 0.381 cm), usually about 0.059 inches (0.1499 cm).
The wall thickness of outer tubular member **406** and inner tubular member **408** can vary from about 0.001 to about 0.005 inches (about 0.00254 to about 0.0127 cm), typically about 0.003 inches (0.00762 cm).
The inner tubular member 408 typically has an outer diameter of about 0.04 to about 0.15 inches (about 0.1016 to about 0.381 cm), usually about 0.054 inches (0.13716 cm) and an inner diameter of about 0.03 to about 0.14 inches (about 0.0762 to about 0.3556 cm), usually about 0.048 inches (0.12192 cm).

The overall working length of catheter **400** may range from about 50 to about 150 cm, typically about 110 to about 120 cm. Preferably, inflation balloon **414** has a total length about 5 to about 20 mm; a working length of about 1 to about 18 mm, preferably about 4 to about 8 mm. Inflation balloon **414** has an inflated working outer diameter of about 4 to about 20 mm, preferably about 4 to about 8 mm within a working pressure range of inflation balloon **414.** In preferred embodiment, outer tubular member **406** and inner tubular member **408** is braided polyimide tubular member from IWG High Performance Conductors. Specifically, the braided polyimide tubular member comprises braided stainless steel, with the braid comprising rectangular or round stainless steel wires. Preferably, the braided stainless steel has about 90 picks per inch. The individual stainless steel strands may be coated with heat resistant polyimide and then braided or otherwise formed into a tubular member or the stainless steel wires or strands may be braided or otherwise formed into a tubular product and the braided surfaces of the tubular product may be coated with a heat resistant polyimide.

As will be appreciated by those skilled in the art, the catheters and generators of the present invention can be used to heat one or more target lung tissue to treat a variety of lung diseases and conditions, including but not limited to: lung tumors, solitary pulmonary nodules, lung abscesses, tuberculosis, as well as a variety of other diseases and disorders. In one instance, a procedure for inducing lung volume reduction (as a treatment for emphysema) involves advancing catheter **400** into a segmental or sub-segmental airway and delivering a controlled vapor dose. As will be appreciated by those skilled in the art, the vapor carries most of the energy and heat required to convert liquid in vapor generator from a liquid into a vapor. Upon dispersion of the vapor into the airways, the vapor penetrates into the interstitial channels between the cells, and distributes thermal area over a relatively large volume of tissue, permitting tissue heating to be accomplished quickly, usually with a few seconds or minutes. Vapor heating of target lung tissue is intended to cause tissue injury, shrinkage and/or ablation, in order to cause volumetric reduction of one or more target lung tissues. Lung volume reduction is immediate and/or occurs over several weeks or months.

Depending on the extent of the volumetric reduction (complete or partial reduction of a lobe) desired, catheter **400** is navigated into one or more airways, preferably as into the segmental or sub-segmental airways and the vapor delivered into as many airway as need during a single procedure to effect the therapeutically optimal extent of lung volume reduction. In a preferred embodiment, a vapor generator configured to create a vapor having a vapor pressure between about 5 - 50 psig (1.34 - 4.40 atm or 135799 - 446063 Pa), at a temperature between about 100°-175° degrees C within vapor generator **300** is employed. The vapor catheter is delivered into the sub-segmental airways that communicate with either the left and right upper lobes, and vapor delivered for a period of 1-20 seconds in each of these airways, to effect volumetric reduction of the left and right upper lobes. Preferably, energy deliver to a target lung tissue is achieved without attendant plural heating sufficient to cause damage to the pleura or a pneumothoraces.

As will be appreciated by one skilled in the art, various imaging techniques (in addition to or in lieu of conventional bronchoscopic imaging) can be employed before, during and after a vapor treatment procedure. Real time fluoroscopy can be used to confirm depth of catheter **400** inside a patient's lung as well as confirm position of catheter in a desired airway. In yet another embodiment, real-time CT guided electromagnetic navigational systems, such as the SuperDimension®/Bronchus system can be employed to accurately guide catheters of the present invention to the desired tissues targets, especially to get the catheters close to target tissues that are peripherally located. In one embodiment of the invention, the present invention can be adapted to work through a working channel of a locatable guide or guide catheter of the SuperDimension CT navigational system.

A medical kit for performing volumetric vapor heating of one or more target lung tissues which comprises a packaged, sterile liquid or liquid composition and a high temperature vapor delivery catheter. Other embodiments of said medical kits can comprise instructions of use, syringes, and the like.

The invention has been discussed in terms of certain embodiments. One of skill in the art, however, will recognize that various modifications may be made without departing from the scope of the invention. For example, numerous variations, changes, and substitutions will now occur to those skilled in the art without departing from the invention. Moreover, while certain features may be shown or discussed in relation to a particular embodiment, such individual features may be used on the various other embodiments of the invention. In addition, while not provided, other energy modalities can be employed for volumetric heating of target lung tissue and its understood that in conjunction with or instead of vapor, such as modalities such as RF, laser, microwave, cryogenic fluid, a resistive heating source, ultrasound and other energy delivery mechanisms can be employed for heating a target lung volume.

## Claims

1. A patient lung volume reduction system, comprising:
a water vapor generator (300);
a vapor delivery catheter (200, 400) having a proximal end adapted to communicate with the water vapor generator and a distal end adapted to be placed within a lumen of a patient's lung;
a vapor circuit (314) communicating with the vapor generator and the catheter;
a priming line (330) communicating with the vapor circuit; and
one or more control valves (320) adapted to direct water vapor from the vapor circuit into the priming circuit and into the catheter;
wherein the water vapor generator comprises:
a controller constructed and arranged to control the generation of a controlled dose of water vapor at a pressure of 5-50 psig (1.34 - 4.40 atm; 135799 - 446063 Pa) and at a temperature in the range of 100-175°C for 1-20 seconds from a location external to the patient for delivery by the catheter into a segmental or subsegmental airway of the patient's lung; and
a user interface (360) for providing a visual readout of vapor delivery duration.

2. The system of claim 1, wherein the distal end of the catheter (200, 400) is adapted to be placed within the lumen through a working channel of a bronchoscope.

3. The system of claim 1, wherein the catheter further comprises a vapor exit port (212, 424) and an occlusion balloon (209, 414) disposed proximal to the vapor exit port.

4. The system of claim 3, wherein the catheter further comprises a balloon inflation lumen (412) and a vapor delivery lumen (410).

5. The system of claim 4, wherein the vapor delivery lumen (410) is concentric with the balloon inflation lumen (412).

6. The system of claim 1, wherein the flow controller comprises a control valve (320).

7. The system of claim 1, wherein the priming line (330) terminates at a vapor collection unit.

## Patentansprüche

1. Patienten-Lungenvolumenreduktionssystem, umfassend:
einen Wasserdampfgenerator (300);
einen Dampfzuführungskatheter (200, 400), der ein proximales Ende, das angepasst ist, um mit dem Wasserdampfgenerator zu kommunizieren, und ein distales Ende, das angepasst ist, um in einem Lumen einer Patientenlunge platziert zu werden, hat;
einen Dampfkreislauf (314), der mit dem Dampfgenerator und dem Katheter kommuniziert;
eine Grundleitung (priming line) (330), die mit dem Dampfkreislauf kommuniziert, und
ein oder mehrere Kontrollventil(e) (320), angepasst, um Wasserdampf aus dem Dampfkreislauf in die Grundleitung und in den Katheter zu lenken;
wobei der Wasserdampfgenerator umfasst:
eine Steuereinrichtung, die konstruiert und angeordnet ist, um die Erzeugung einer kontrollierten Dosis an Wasserdampf mit einem Druck von 5 - 50 psig (1,34 - 4,40 atm; 135.799 - 446.063 Pa) und mit einer Temperatur im Bereich von 100-175°C für 1-20 Sekunden von einer Stelle aus, die zu dem Patienten extern ist, zur Zuführung durch den Katheter in einen segmentalen oder subsegmentalen Atemweg der Lunge des Patienten zu steuern, und
eine Benutzerschnittstelle (360) zur Bereitstellung eines visuellen Ablesens der Dampfzuführungsdauer.

2. System gemäß Anspruch 1, wobei das distale Ende des Katheters (200, 400) angepasst ist, um durch einen Arbeitskanal eines Bronchoskops in dem Lumen platziert zu werden.

3. System gemäß Anspruch 1, wobei der Katheter außerdem eine Dampfaustrittsöffnung (212, 424) und einen Okklusionsballon (209, 414), der proximal zu der Dampfaustrittsöffnung angeordnet ist, umfasst.

4. System gemäß Anspruch 3, wobei der Katheter außerdem ein Balloninflationslumen (412) und ein Dampfzuführungslumen (410) umfasst.

5. System gemäß Anspruch 4, wobei das Dampfzuführungslumen (410) mit dem Balloninflationslumen (412) konzentrisch ist.

6. System gemäß Anspruch 1, wobei die Strömungssteuereinrichtung ein Kontrollventil (320) umfasst.

7. System gemäß Anspruch 1, wobei die Grundleitung (330) an einer Dampfsammeleinheit endet.

## Revendications

1. Système de réduction du volume pulmonaire d'un patient, comprenant :
un générateur de vapeur d'eau (300) ;
un cathéter de distribution de vapeur (200, 400) ayant une extrémité proximale adaptée pour communiquer avec le générateur de vapeur d'eau et une extrémité distale adaptée pour être placée à l'intérieur d'une lumière du poumon d'un patient ;
un circuit de vapeur (314) communiquant avec le générateur de vapeur et le cathéter ;
une ligne d'amorçage (330) communiquant avec le circuit de vapeur ; et
un ou plusieurs clapets de commande (320) adaptés pour diriger de la vapeur d'eau du circuit de vapeur dans le circuit d'amorçage et dans le cathéter ;
dans lequel le générateur de vapeur d'eau comprend :
un dispositif de commande conçu et disposé de manière à commander la formation d'une dose réglée de vapeur d'eau à une pression manométrique de 5 - 50 psi (1,34 - 4,40 atm ; 135799 - 446063 Pa) et à une température de l'ordre de 100 à 175°C pendant 1 à 20 secondes depuis un emplacement extérieur au patient destinée à être délivrée par le cathéter dans une voie segmentaire ou sous-segmentaire du poumon du patient ; et
une interface utilisateur (360) destinée à fournir une indication visuelle de la durée de l'administration de vapeur.

2. Système selon la revendication 1, dans lequel l'extrémité distale du cathéter (200, 400) est adaptée pour être placée à l'intérieur de la lumière à travers un canal opérateur d'un bronchoscope.

3. Système selon la revendication 1, dans lequel le cathéter comprend en outre un orifice de sortie de vapeur (212, 424) et un ballonnet d'occlusion (209, 414) disposé de façon proximale par rapport à l'orifice de sortie de vapeur.

4. Système selon la revendication 3, dans lequel le cathéter comprend en outre une lumière de gonflage de ballonnet (412) et une lumière de distribution de vapeur (410).

5. Système selon la revendication 4, dans lequel la lumière de distribution de vapeur (410) est concentrique à la lumière de gonflage de ballonnet (412).

6. Système selon la revendication 1, dans lequel le dispositif de commande de débit comprend un clapet de commande (320).

7. Système selon la revendication 1, dans lequel la ligne d'amorçage (330) se termine au niveau d'une unité de collecte de vapeur.
